# EUROPEAN PATENT APPLICATION

(11) **EP 3 363 448 A1**
(43) Date of publication of application: **22.08.2018**
(21) Application number: 18155228.2
(22) Date of filing: 06.02.2018
(51) Int. Cl.: A61K 36/22, A61P 1/00, A61P 1/04, A61P 35/00

(54) **TERPENE ENRICHED FRACTIONS FREE FROM POLYTERPENES EXTRACTED FROM CHIOS MASTIC GUM AND COSMETIC, NUTRACEUTICAL, MEDICAL DEVICES AND PHARMACEUTICAL COMPOSITIONS CONTAINING THEM**

(30) Priority: 15.02.2017 IT 201700016726
(71) Applicant: Phytoitalia SRL, 20124 Milano (IT)
(72) Inventor: VOLPI, Nicola, 41125 MODENA (IT); ABBIATI, Ezio, 25081 BEDIZZOLE (BS) (IT)
(74) Representative: Bianchetti Bracco Minoja S.r.l.

(57) **Abstract**

The invention relates to isolated fractions of Chios mastic gum enriched in terpene monomers and oligomers and substantially free of poly-terpene polymers with a degree of polymerization greater than 8 and a molecular weight in the range from about 1,000 to about 100,000 as well as to a process for their preparation. The invention also relates to compositions comprising an isolated fraction of monomeric and oligomeric terpenes extracted and purified by reverse-phase chromatography and essentially free of polymers. The monomeric and oligomeric enriched terpenes fraction obtained from Chios mastic gum exhibits biological activities in cosmetic, nutraceutical, medical devices and pharmaceutical applications.

## Description

### SUMMARY

The invention relates to isolated fractions of Chios mastic gum enriched in terpene monomers and oligomers and free of poly-terpene polymers with a degree of polymerization greater than 8 and an average molecular weight in the range from 1,000 to 100,000 as well as to a process for their preparation. The invention also relates to compositions comprising an isolated fraction of monomeric and oligomeric terpenes extracted and purified by reverse-phase chromatography, free of polymers and having a purity degree higher than 98%. The monomeric and oligomeric enriched terpenes fraction obtained from Chios mastic gum exhibits biological activities in cosmetic, nutraceutical, medical devices and pharmaceutical applications.

### BACKGROUND OF THE INVENTION

Chios mastic gum is a resin generated by the plant *Pistacia lentiscus* var. chia, generally cultivated in Mediterranean countries and particularly in the southern part of the Greek island of Chios. *P. lentiscus* is a very ancient plant and the related gum has been used since many centuries. In fact, the oldest reference to mastic gum has been traced by Herodotes in the 5th Century B.C.. Greeks have used Chios mastic gum in diverse applications, such as cooking, preparation of beverages, cosmetics and paints, and for treatment of gastric diseases [1]. Recent studies have associated specific pharmaceutical properties of Chios mastic gum with its particular molecular components [2].

Chios mastic gum is a resin having a teardrop-like appearance on the stem and branches of the plant forming at sites of incisions. Initially, Chios mastic gum is secreted as a colloidal clear liquid that solidifies after 15-20 days at room temperature acquiring an opaque crystalline appearance similar to a stalactite characterized by a balsam-like odour. This material is collected and further processed. The hardness of the Chios mastic gum depends on several factors, as well as the temperature, exposure time in nature and its size.

Increasing scientific evidences are available on the therapeutic activity of Chios mastic gum. Its gastro-intestinal, antioxidant, anti-inflammatory, antidiabetic, antimicrobial and anticancer activity, as well as its beneficial effects in oral hygiene and in skin care are largely documented [2]. In particular, it is used as a seasoning in Mediterranean cuisine, in the production of chewing gum, in perfumery, in dentistry, and for the relief of epigastric pain and protection against peptic ulcer [2].

Chios mastic gum is largely insoluble in water and soluble in different organic solvents, including methanol, dimethyl-sulfoxide (DMSO), acetone and chloroform. Non-polar constituents have been analyzed and the composition of the various extracts from Chios mastic gum, the so-called Chios mastic gum-derived mastic gum oil, has been characterized and several constituents isolated and identified in various fractions [3-9]. Up to more than 70 constituents of Chios mastic gum have been found and more than 60 have been identified. Six components, namely α-pipene, β-pipene, β-myrcene, linalool, *trans*-caryophyllene and camphene, account for 65% to 80% of the weight of the Chios mastic gum.

The biological activities of Chios mastic gum can be attributed to various compounds. The active components contributing to its therapeutic effects belong to the class of terpenes (mono- and sesquiterpenoids, triterpenic acids and triterpenoids) [8]. Examples of said components include oleanonic acid, moronic acid, 24*Z*-masticadienonic acid, 24*Z*-isomasticadienonic acid, 24Z-masticadienolic acid, 24Z-isomasticadienolic acid, tirucallol, dammaradienone, 28-norolean-12-en-3-one, oleanonic aldehyde and oleanolic aldehyde.

Chios mastic gum contains polymeric β-myrcene that has not only therapeutic benefit [8], but even is detrimental to certain biological activities attributed to crude mastic gum extracts and preparations.

Terpenes are only one of the largest classes of plant natural products, with more than 20,000 different species reported. The vast majority of terpene diversity occurs in the plant kingdom, although other organisms also produce this kind of molecules. More than 100 different terpene scaffolds are currently known in plants. Nature's terpene reservoir remains largely undiscovered, despite the considerable commercial and therapeutic interest in these compounds for a range of applications [10].

Triterpenic acids, in particular, possess various biological capacities such as anti-inflammatory, antioxidant, antiatherogenic, antihyperlipidemic, anti-tumor, antidiabetic and hepatoprotective effects [11]. Chios mastic gum has been demonstrated to contain many of these active molecules such as oleanonic acid, moronic acid, 24*Z*-masticadienonic acid, 24*Z*-isomasticadienonic acid, 24*Z-*masticadienolic acid, 24*Z*-isomasticadienolic acid [2].

GR 1,003,541 discloses antimicrobial and antifungal action of the Chios mastic oil extracted from the leaves, branches and fruit of Pistacia lentiscus.GR 1,003,868 discloses the use of a product derived from the same plant as antioxidant, wound healing inductor and cytostatic agent.

US 2005/0238740 discloses that various fractions extracted from mastic resin possess anti-microbial and cell anti-proliferative activities. A first extract (termed "total fraction" or "whole extract") contains all the compounds of the mastic gum except the polymer resin. A second extract is an acid fraction containing all the triterpenic acids of the total fraction, and a third extract is a neutral fraction containing all the other terpenes of the total fraction.

EP 1520585 discloses use of essential oils distilled from different parts of Pistacia species containing a large number of monomeric terpene compounds in varying proportions possessing activity against tumor cells lines.

WO 2005/112967 discloses the purification from Chios mastic of an anti-proliferative compound found in a soluble fraction obtained by suspending mastic in a solvent selected from a non-acidic, aliphatic hydrocarbon, an aqueous solution containing at least 25% of a water-soluble, non-acidic, aliphatic hydrocarbon or a combination and removing the insoluble fraction.

US 5,506,406 discloses mastic oil produced by dissolving mastic in oil or fat environment with further optionally adding an amphipathic substance such as chitin or chitosan being effective for eliminating and inhibiting Helicobacter pylori and for reducing the smell of feces.

US. 5,637,290 discloses an oral hygiene product comprising the combination of a toothpaste and an ingredient selected from natural mastic, extracted mastic oil and synthetic mastic oil agents.

US 6,623,728 discloses cosmetic skin care emulsion compositions comprising from about 0.001% to about 10% solubilized gum mastic, a volatile water miscible solvent and a cosmetically acceptable vehicle. According to the disclosure, solvents including ethanol, methanol, propanol, isopropyl alcohol and mixtures are used to obtain the solubilized gum mastic.

US 6,811,769 discloses an oral composition comprising an oil extract of mastic prepared with olive oil or palm oil having antibacterial action against periodontal disease-related bacteria and tooth decay-related bacteria.

Paraschos et al. [8] reported a preparation of a total mastic extract free from polymers prepared by polar solvent extraction of crude Chios mastic and removal of the insoluble poly-β-myrcene, further separated in acidic and neutral fractions by means of liquid/liquid partition with organic solvents and neutral and acidic aqueous solutions. According to the publication, administration of the polymer-free extract to mice infected Helicobacter pylori resulted in a 30-fold reduction of bacterial colonization, largely attributable to a particular compound purified from the acid fraction. The authors state that the polymer-free extract was prepared since the high percentage of poly-β-myrcene in crude mastic preparations was speculated to hinder potential *in vivo* activity during oral administration. The authors further state that removal of the poly-β-myrcene enhances the anti-Helicobacter pylori activity. No mention is made of other polymers found in Chios mastic having a polymerization degree higher than 8 and a molecular weight ranging from 1000 to 100.000. WO 2010100651 relates to compositions comprising an isolated fraction of polymeric myrcene from mastic gum and formulations which maintain the biological activity of the active polymer.

The prior art therefore does not teach or suggest any fractionation of the entire extract of Chios mastic gum by means of hydrophobic resins and reverse-phase chromatography to purify and enrich the active terpenes component and eliminate the poly-β-myrcene polymer and other polymers from the Chios mastic gum.

### DESCRIPTION OF THE INVENTION

It has now been found that a fraction consisting of all the terpene present in Chios mastic and substantially free of poly-beta-myrcene and of other polymeric terpenes may be conveniently obtained by subjecting the crude mastic to a simple and convenient reverse-phase chromatographic procedure.

Said procedure turned out to be more efficient than the prior art methods based on extraction with solvents or liquid-liquid partition techniques.

The obtained fraction comprises both the neutral terpenes and the triterpenic acid, in addition to small oligomeric forms of myrcene.

In a first embodiment, the invention provides therefore an isolated fraction of Chios mastic gum enriched in terpene monomers and oligomers and substantially free of poly-terpene polymers with a degree of polymerization greater than 8 and an average molecular weight in the range from about 1,000 to about 100,000.

The isolated fraction of the invention is characterized by a content of terpene monomers and oligomers higher than 98% by weight.

The isolated fraction contains hemiterpenes, monoterpenes, sesquiterpenes, diterpenes, sesterterpenes, terpenes triterpenes, in their neutral and acidic forms, sesquarterpenes, and tetraterpenes, containing eight isoprene units. The isolated active fraction of the invention also contains myrcene monomers and myrcene oligomeric forms having a degree of polymerization of less than 8.

The invention also concerns a process for the preparation of the terpene fractions, comprising the steps of:
(a) treating Chios mastic gum with methanol or a mixture of methanol and ethyl acetate;
(b) subjecting the solution obtained in a) to chromatography on a reverse-phase resin and eluting with methanol to give a fraction rich in terpenes monomers and oligomers;
(c) regenerating the reverse-phase resin with ethyl acetate;
(d) removing methanol from the soluble fraction obtained in step (b).

The invention also provides pharmaceutical and nutraceutical compositions comprising the fractions obtained by the above-mentioned process. The composition of the invention may be useful for a variety of therapeutic applications.

### DETAILED DESCRIPTION OF THE INVENTION

As used herein, the term terpenes encompasses a very large heterogeneous family of compounds comprising hemiterpenes, consisting of a single isoprene unit, monoterpenes, consisting of two isoprene units, sesquiterpenes, consisting of three isoprene units, diterpenes, consisting of four isoprene units, sesterterpenes, terpenes having 25 carbons and five isoprene units, triterpenes, consisting of six isoprene units in their neutral and acidic forms (the so-called triterpenic acids), sesquarterpenes, consisting of seven isoprene units, tetraterpenes, containing eight isoprene units. The isolated fraction of the invention also contains myrcene monomers and myrcene oligomeric forms having a degree of polymerization less than 8.

As used herein, the term "degree of purity" refers to the content of the active terpenes, comprising hemiterpenes, monoterpenes, sesquiterpenes, diterpenes, sesterterpenes, triterpenes, in their neutral and acidic forms, sesquarterpenes, tetraterpenes, expressed as a percentage by weight per weight basis.

The process comprises the steps of:
(a) treating mastic gum with methanol or a mix of methanol and ethyl acetate;
(b) preparing a chromatography column with a reverse-phase resin;
(c) charging the column with the solution obtained in step (a);
(d) eluting with 100% methanol a fraction rich in terpenes monomers and oligomers from the reverse-phase chromatography;
(e) regenerating the column by removing the column the fraction rich in poly-terpenes polymers with 100% ethyl acetate;
(f) removing the solvent from the soluble fraction obtained in step (d), for instance by a means of rotary evaporation or equivalent methods, lyophilization or a combination thereof.

The fraction obtained in step (d) is substantially rich in terpenes monomers and oligomers having a degree of polymerization less than 8, are soluble in methanol and insoluble in ethyl acetate. Terpenes belong to the families of hemiterpenes, monoterpenes, sesquiterpenes, diterpenes, sesterterpenes, triterpenes, in their neutral and acidic forms (the so-called triterpenic acids), sesquarterpenes and tetraterpenes.

The composition of the fraction obtained in step (d) is substantially free from poly-terpenes polymers that are insoluble in methanol and soluble in ethyl acetate.

As an alternative to Chios mastic gum from Pistacia lentiscus, other gum mastics from a plant of the Anacardiaceae family may be used as a starting material, in particular from Pinus, Picea, Juniperus, Alsies, Larix, Antirrhinum, Boswellia, Citrus and Gynura. Suitable Pistacia species include Pistacia atlantica, Pistacia palestina, Pistacia saportae, Pistacia terebinthus, Pistacia vera and Pistacia integerrima.

The process of the invention is disclosed in detail hereinbelow:

### STEP 1. Dissolution of Chios mastic gum raw material in an appropriate solvent

Chios mastic gum raw material is dissolved in a mixture 40% ethyl acetate/60% methanol or 100% ethyl acetate, preferably, in 80% ethyl acetate/20% methanol.
i. Chios mastic gum raw material is dissolved in the solvent of point (i) at a concentration weight/volume from 1 mg/mL to 50 mg/mL, typically at a concentration of 10 mg/mL.
ii. The solution of Chios mastic gum of point (ii) is mixed under stirring at a temperature from 15°C to 25°C, preferably from 20°C to 23°C. The solution is stirred for a period from 6 to 12 hours, preferably from 8 to 10 hours. The mastic raw material is preferably dissolved in an ultrasound bath at a controlled temperature and with a power from 50 to 150 Watts, preferably 100 Watts.
iii. The solution at point (iii) is centrifuged to remove the insoluble material at between 5.000 and 10.000 g, preferably 7.000 g, at a temperature from 18°C to 25°C, preferably 20°C, for 30 and 90 min, preferably 60 min. The solution at point (iii) may be filtered or further filtered after centrifugation on cellulose filters at a temperature from 18°C to 25°C, preferably 20°C.

### STEP 2. Fractionation on reverse-phase chromatography

i. Chromatographic columns are prepared by packing reverse-phase resins available on the market, preferably BioSil C18 HL90 40-63 and Sephadex LH20 resins.
ii. The resins of point (i) are gently dissolved in a solution comprising 15%-30% methanol, preferably at 20% methanol. Resins in solution are gently mixed at room temperature for 30-120 min, depending on the type of resin. The solution with dissolved resins is degassed in a ultrasound bath for 10-20 min and the columns are gently packed.
iii. The volume of the columns and the amount of reverse-phase resin in point (ii) is strictly related to the concentration of the solution obtained at STEP 1. Preferred amount of resin and concentration of the solution ratios are between 10 mg/mL of solution/10 mg of resin up to 30 mg/mL of solution/10 mg of resin.
iv. The solution prepared in STEP 1 is charged on the top of the column packed with the resin and the recovered solution is recharged on the same column. The recovered solution obtained by the second elution is discarded. All material extracted from the Chios mastic by the procedure reported in STEP 1 composed of the active fraction of terpenes but also poly-terpenes, in particular the poly-β-myrcene polymer, are now adsorbed on the hydrophobic groups of the resin.
v. A solution of 100% methanol is added on the column prepared according to point (iii). In a preferred embodiment, 10 mL of methanol are added for 10 mg of resin and the eluted solution recovered and analyzed for the presence of the active terpenes fraction and poly-terpenes polymers.
vi. A solution of 100% ethyl acetate is now added on the column after treatment as illustrated at point (v). In a preferred embodiment, 20 mL of ethyl acetate are added for 10 mg of resin and the eluted solution recovered and analyzed for the presence of the active terpenes and poly-terpenes polymers.

### STEP 3. Drying of the solution rich in terpenes active fraction

i. The solution of STEP 2 in methanol is dried by means of a rotary evaporator at a temperature ranging between 30°C and 50°C, preferably at 40°C, as higher temperatures induce a degradation of terpenes, or lyophilized. In a favorite embodiment, lyophilization is used.

The two solutions obtained in the STEP 2, eluted with 100% methanol and 100% ethyl acetate, have been tested by HPLC-ESI-MS (electrospray ionization mass) for the content and presence of terpenes, and by HPSEC (high-performance size-exclusion chromatography) for the content and presence of poly-terpenes polymers.

HPLC-ESI-MS by using reverse-phase analytical columns is able to separate terpenes based on their structure and hydrophobicity and detected by ESI-MS. Moreover, ESI-MS and tandem mass is able to identify the different molecules based on their mass values and fragmentation pattern.

As exhaustively described in the examples, the fraction eluted from the column packed with reverse-phase resins and constituted of 100% methanol, exclusively contains terpenes, with a title greater than 98% and myrcene monomers and oligomers having a degree of polymerization less than 8 and devoid in poly-terpenes with a polymerization degree in the range from 8 to abot 1800 and a molecular weight in the range from 1,000 to 100,000.

The fraction eluted from the column with 100% ethyl acetate exclusively contains poly-terpenes with a degree of polymerization in the range from 8 to 1800 and a molecular weight in the range from 1,000 to 100,000, confirming that a preferential separation has been obtained by using methanol and ethyl acetate.

The fractions obtained by the above described process may be suitably formulated, in admixture with an acceptable carrier in a form suitable for administration by oral, topical, parenteral or transdermal route, for cosmetic, dermatologic, pharmaceutical or nutraceutical applications.

A topical formulation may be suited for the dermal, vaginal, rectal, inhalation, intranasal, ocular, auricular or buccal administration. Examples of suitable carriers include a mineral oil or a vegetable oil or combination thereof. The carrier may further comprise at least one wax, such as beeswax, vegetable waxes, sugar cane waxes, mineral waxes and synthetic waxes.

Examples of suitable forms include capsules, tablets, liposomes, asuppositories, a suspensions, an ointments, creams, lotions, solutions, emulsions, nanoemulsions, microemulsions, films, cements, powder, glues, aerosols, sprays.

The pharmaceutical or nutraceutical compositions of the invention may be manufactured by means of conventional mixing, granulating, softgel encapsulation, dissolving, extracting or lyophilizing processes.

The invention is described in greater detail in the following examples.

### Example 1

Chios mastic gum raw material having a crystalline appearance is dissolved at a concentration of 20 mg/mL in 100% ethyl acetate. The solution is mixed under a stirrer mix at a temperature of 20°C for 8 hours in an ultrasound bath with a power of 100 Watts.

After this, the resulting solution is centrifuged to remove all insoluble material at 7.000 g at 20°C for 60 min.

A Chromatographic column is prepared by packing reverse-phase resin BioSil C18 HL90 40-63 in 20% methanol by gently mixing at the room temperature of 22°C for 30 min. After degassing in an ultrasound bath for 10 min, the resin is gently packed in the column by simple gravity. 10 mg of resin are used and packed in a column of 1 cm x 20 cm.

The solution of 20 mg/mL of Chios mastic gum in 100% ethyl acetate is charged on the top of the column and the recovered solution is recharged on the same column just one time. After this step, the recovered solution obtained by the second elution is discarded.

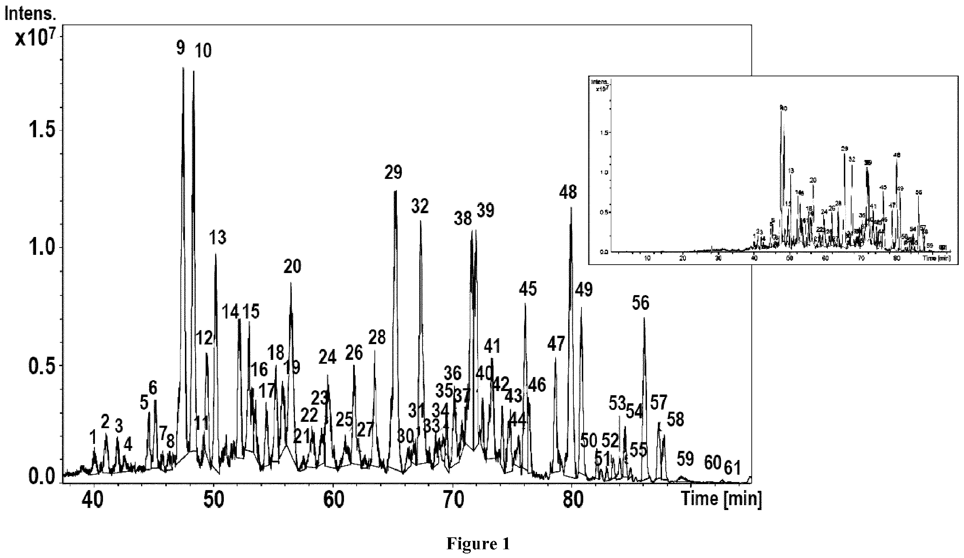

10 mL of methanol are added on the column and the eluted solution is recovered and analyzed for the presence of the active terpenes fraction and poly-terpenes polymers (Figure 1 and Table 1).

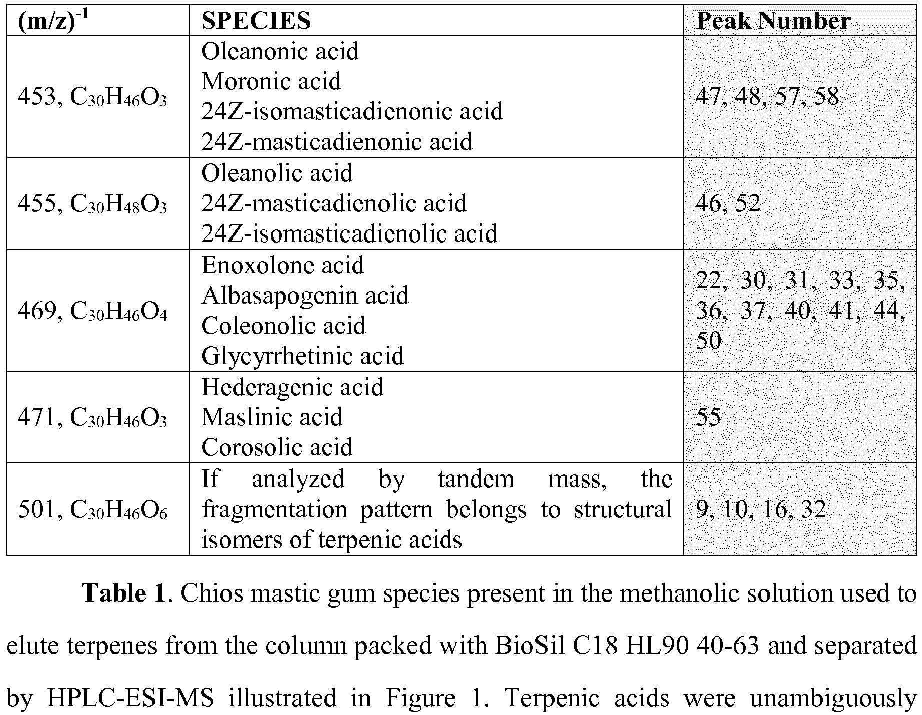

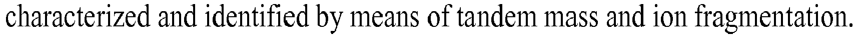

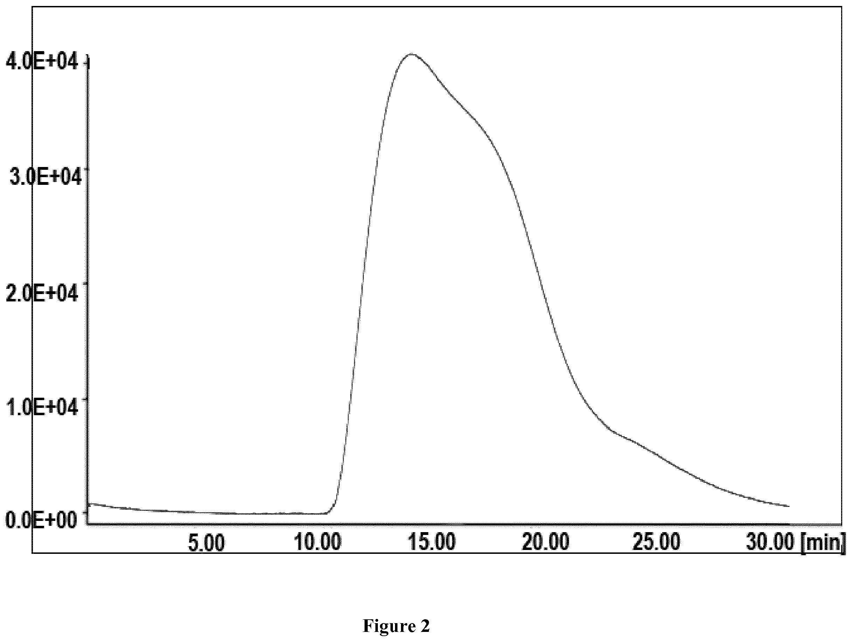

20 mL of ethyl acetate are then added to the column and the eluted solution recovered and analyzed for the presence of the active terpenes and poly-terpenes polymers (Figure 2).

The solution obtained by eluting with methanol is dried by using a rotary evaporator at a temperature of 40°C. 7.6 mg of material is obtained in dry form, with a recovery of about 38%.

Figure 1 illustrates the HPLC-ESI-MS chromatogram of the solution of methanol used to elute terpenes from the column packed with BioSil C18 HL90 40-63. As evident, many species are present having low-molecular weight with a degree of polymerization less than 8. By using mass values and tandem mass with the pattern of fragmentation, many terpenes species belonging to hemiterpenes and/or monoterpenes and/or sesquiterpenes and/or diterpenes and/or sesterterpenes and/or triterpenes, in their neutral and acidic forms, and/or sesquarterpenes and/or tetraterpenes, were identified (Table 1). Moreover, in detail, the procedure object of the present invention was able to enrich the active fraction with terpenic acid considered to be the most active component against *Helicobacter pylori* [8] (Table 1).

On the contrary, the solution eluted with ethyl acetate was found to lack in these species with a resulting HPLC-ESI-MS chromatogram showing no peak (not shown).

HPSEC was used to verify the presence of poly-terpene polymers with a degree of polymerization greater than 8 and a molecular weight in the range from 1,000 to 100,000. No peak was detected in the methanol solution (not shown) giving us a title in terpenes of 99.2%, contrary to ethyl acetate extract showing a large peak belonging to poly-terpenes having a degree of polymerization comprises between about 10 and 500 (Figure 2).

HPSEC was performed by using two stainless steel columns (TSKgel of PW type, G6000 and G5000, 17 *µ*m particle size by Tosoh Bioscience, both 7.8 mm × 30 cm) connected in series with a guard precolumn, separation temperature at 35°C with a mobile phase of 0.15 M NaCl solution at a flow-rate of 0.4 ml/min.

In conclusion, the methanol solution obtained from the column packed with BioSil C18 HL90 40-63 was essentially composed of terpene monomers and oligomers with a degree of polymerization lower than about 8 with a purity greater than 98%. Furthermore, the recovered methanol solution shows the presence of a great content of terpenic acid considered to be the most active component against *Helicobacter pylori* [8]. The poly-terpene polymers present in Chios mastic are all recovered in 100% ethyl acetate solution.

### Example 2

Chios mastic gum raw material having a crystalline appearance is dissolved at a concentration of 30 mg/mL in 50% methanol and 50% ethyl acetate. The solution is mixed under a stirrer mix at a temperature of 25°C for 10 hours in an ultrasound bath with a power of 150 Watts.

After this, the resulting solution is centrifuged to remove all insoluble material in a centrifuge at a force of 10.000 g at 22°C for 90 min and further filtered on filters of cellulose filters at a temperature of 22°C.

A Chromatographic column is prepared by packing reverse-phase resin Sephadex LH20 in 25% methanol by gently mixing at the room temperature of 24°C for 60 min. After degassing in an ultrasound bath for 10 min, the resin is gently packed in the column by simple gravity. 10 mg of resin are used and packed in a column of 1 cm x 20 cm.

The solution of 30 mg/mL of Chios mastic gum in 50% methanol and 50% ethyl acetate is charged on the top of the column and the recovered solution is recharged on the same column just one time. After this step, the recovered solution obtained by the second elution is discarded.

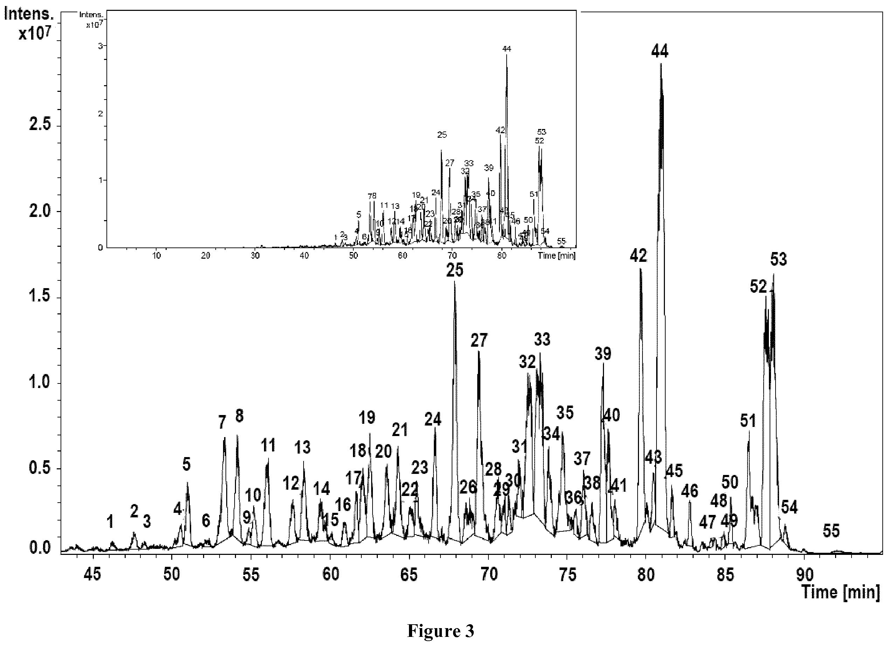

10 mL of methanol are added on the column and the eluted solution is recovered and analyzed for the presence of the active terpenes fraction and poly-terpenes polymers (Figure 3 and Table 2).

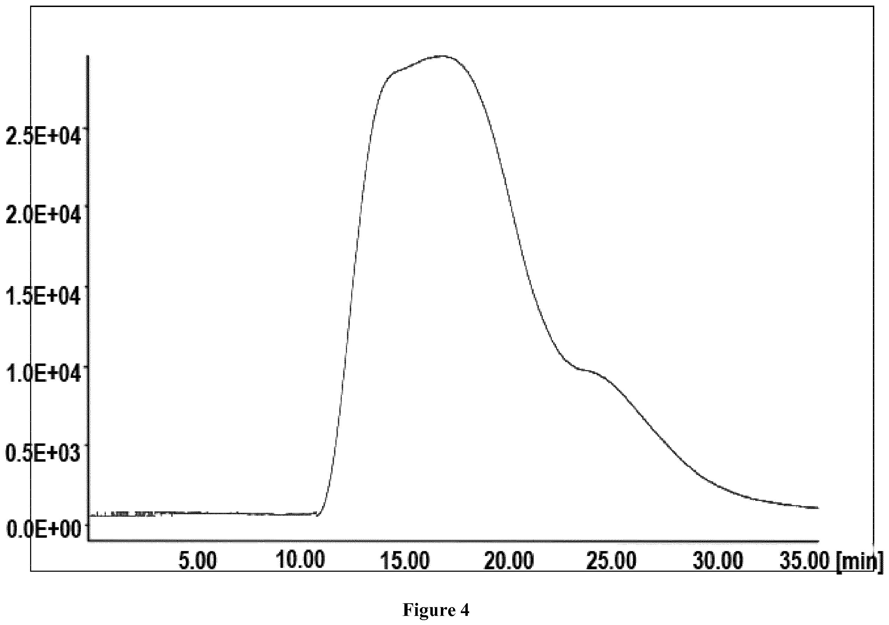

20 mL of ethyl acetate are then added to the column and the eluted solution recovered and analyzed for the presence of the active terpenes and poly-terpenes polymers (Figure 4).

The solution obtained by eluting with methanol is lyophilized.

Figure 3 illustrates the HPLC-ESI-MS chromatogram of the solution of methanol used to elute terpenes from the column packed with Sephadex LH20. Also by using this different resin compared to Example 1, many species are present having low-molecular weight with a degree of polymerization less than 8. By using mass values and tandem mass with the pattern of fragmentation, many terpenes species belonging to hemiterpenes and/or monoterpenes and/or sesquiterpenes and/or diterpenes and/or sesterterpenes and/or triterpenes, in their neutral and acidic forms, and/or sesquarterpenes and/or tetraterpenes, were identified (Table 2). Moreover, also the procedure adopted in this Example was able to enrich the active fraction with terpenic acid considered to be the most active component against *Helicobacter pylori* [8] (Table 2).

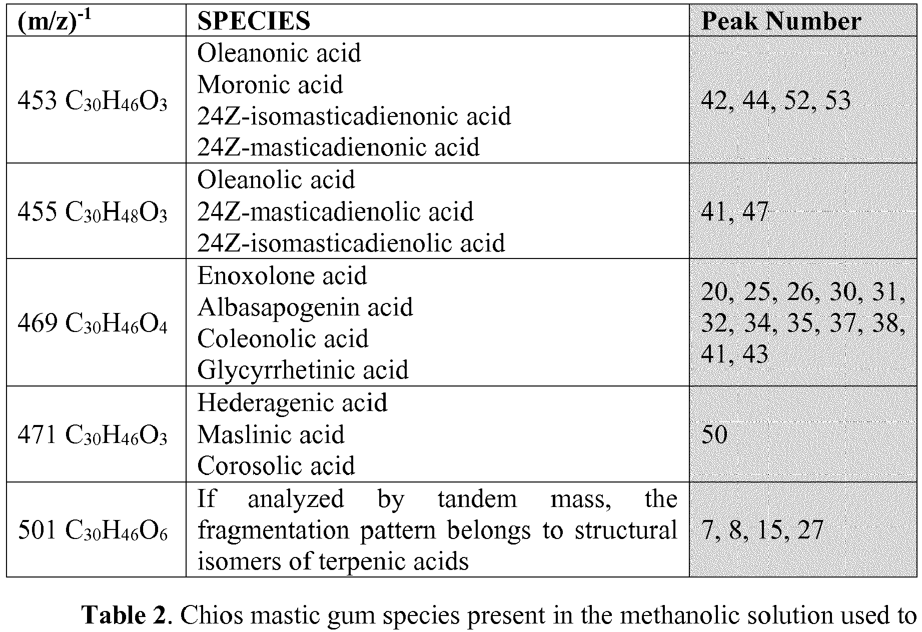

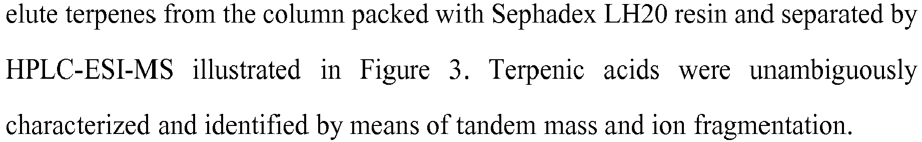

On the contrary, also in this case, the solution eluted with ethyl acetate shows an HPLC-ESI-MS chromatogram with no peak (not shown) confirming the absence of terpene species having low-molecular weight with a degree of polymerization less than 8.

HPSEC was used to verify the presence of poly-terpene polymers with a degree of polymerization greater than 8 and a molecular weight in the range from 1,000 to 100,000. No peak was detected in the methanol solution (not shown) giving a title in terpenes of 98.7%, contrary to ethyl acetate extract showing a large peak belonging to poly-terpenes having a degree of polymerization determined between 15 and 700 (Figure 4).

HPSEC was performed by using two stainless steel columns (TSKgel of PW type, G6000 and G5000, 17 *µ*m particle size by Tosoh Bioscience, both 7.8 mm × 30 cm) connected in series with a guard precolumn, separation temperature at 35°C with a mobile phase of 0.15 M NaCl solution at a flowrate of 0.4 ml/min.

In conclusion, the methanol solution obtained from the column packed with Sephadex LH20 resin described in this Example essentially consists of terpene monomers and oligomers with a degree of polymerization lower than 8 with a purity greater than 98%. Furthermore, the recovered methanol solution shows the presence of an high content of terpenic acid considered to be the most active component against *Helicobacter pylori*. The poly-terpene polymers present in Chios mastic are recovered in 100% ethyl acetate solution.

### REFERENCES

1. Paraschos S, Mitakou S, Skaltsounis AL. Chios gum mastic: A review of its biological activities. Curr Med Chem 19, 2292-302, 2012.
2. Dimas KS, Pantazis P, Ramanujam R. Chios mastic gum: a plant-produced resin exhibiting numerous diverse pharmaceutical and biomedical properties. In Vivo 26, 777-85, 2012.
3. Marner FG, Freyer A, Lex J. Triterpenoids from gum mastic, the resin of Pistacia lentiscus. Phytochemistry 30, 3709-12, 1981.
4. Papageorgiou VP, Mellidis AS, Argyriadou N. The chemical composition of the essential oil of mastic gum. J Essent Oil Res 3, 107-11, 1991.
5. Magiatis P, Melliou E, Skaltsounis AL, Chinou IB, Mitaku S. Chemical composition and antimicrobial activity of the essential oils of Pistacia lentiscus var. chia. Planta Med 65, 749-52, 1999.
6. Duru ME, Cakir, A, Kordali S, Zengin H, Harmandar M, Izumi S, Hirata T. Chemical composition and antifungal properties of essential oils of three Pistacia species. Fitoterapia 74, 170-6, 2003.
7. Koutsoudaki C, Krsek M, Rodger A. Chemical composition and antibacterial activity of the essential oil and the gum of Pistacia lentiscus var. chia. J Agric Food Chem 53, 7681-5, 2005.
8. Paraschos S, Magiatis P, Mitakou S, Petraki K, Kalliaropoulos A, Maragkoudakis P, Mentis A, Sgouras D, Skaltsounis AL. In vitro and in vivo activity of Chios mastic gum extracts and constituents against Helicobacter pylori. Antimicrob Agents Chemother 51, 551-9, 2007.
9. Al-Habbal MJ, Al-Habbal Z and Huwez FU. A double-blind controlled clinical trial of mastic and placebo in the treatment of duodenal ulcer. Clin Exp Pharmacol Physiol 11, 541-4, 1984.
10. Boutanaev AM, Moses T, Zi J, Nelson DR, Mugford ST, Peters RJ, Osbourn A. Investigation of terpene diversification across multiple sequenced plant genomes. Proc Natl Acad Sci USA, 112, E81-8, 2015*.*
11. Zhang S, Sun Y, Sun Z, Wang X, You J, Suo Y. Determination of triterpenic acids in fruits by a novel high performance liquid chromatography method with high sensitivity and specificity. Food Chem 146, 264-9, 2014.

## Claims

1. An isolated fraction of Chios mastic gum (from *Pistacia lentiscus* var. *chia*) enriched in terpene monomers and oligomers and free of poly-terpene polymers with a degree of polymerization greater than 8 and a molecular weight in the range from 1,000 to 100,000.

2. The isolated fraction according to claim 1, wherein the content of terpene monomers and oligomers exceeds 98% by weight.

3. The isolated fraction according to claim 1 or 2, wherein the terpene monomers include terpene acids, hemiterpenes, monoterpenes, sesquiterpenes, diterpenes, sesterterpenes, triterpenes in their neutral or acidic forms, sesquarterpenes and tetraterpenes.

4. The isolated fraction according to claim 3, wherein terpenic acids include oleanonic acid, moronic acid, 24*Z*-masticadienonic acid, 24*Z*-isomasticadienonic acid, 24Z-masticadienolic acid, 24Z-isomasticadienolic acid, tirucallol, dammaradienone, 28-norolean-12-en-3-one, oleanonic aldehyde and oleanolic aldehyde.

5. The isolated fraction according to any one of claims from 1 to 4, having less than 2% by weight of β-myrcene polymer.

6. A process for the preparation of the terpene fractions of claims 1-5, comprising the steps of:
(a) treating Chios mastic gum (from *Pistacia lentiscus* var. *chia*) with methanol or a mixture of methanol and ethyl acetate;
(b) subjecting the solution obtained in a) to chromatography on a reverse-phase resin and eluting with methanol to give a fraction rich in terpenes monomers and oligomers;
(c) regenerating the reverse-phase resin with ethyl acetate;;
(d) removing methanol from the soluble fraction obtained in step (b).

7. A process according to claim 6 wherein methanol in step (d) is removed by evaporation, lyophilization or a combination thereof.

8. A pharmaceutical, cosmetic or nutraceutical composition comprising the fractions of claims 1-5.

9. A composition according to claim 8 in form of capsules, tablets, suppositories, suspensions, ointments, creams, lotions, solutions, emulsions, films, powders, glues, aerosols, sprays.
